# EUROPEAN PATENT APPLICATION

(11) **EP 2 556 843 A1**
(43) Date of publication of application: **13.02.2013**
(21) Application number: 11765327.9
(22) Date of filing: 15.03.2011
(51) Int. Cl.: A61L 27/00

(54) **METHODS FOR MANUFACTURING A REGENERATED TOOTH UNIT**

(30) Priority: 01.09.2010 JP 2010196009; 07.04.2010 JP 2010088967
(71) Applicant: Organ Technologies Inc., Chiyoda-ku Tokyo 101-0048 (JP)
(72) Inventor: TSUJI, Takashi, Tokyo 162-8601 (JP); NAKAO, Kazuhisa, Tokyo 162-8601 (JP); OSHIMA, Masamitsu, Tokyo 162-8601 (JP)
(74) Representative: Stephen, Robert John
(86) International application number: PCT/JP2011/056007
(87) International publication number: WO 2011/125425

(57) **Abstract**

Problem to be Solved

The object of the present invention is to provide a method for culturing a regenerated tooth germ so that the shape of the tooth is not restricted by pressure or space even when cultured in vivo and the extension of the tooth can also be controlled. It is also an object to provide a method which allows transplantation of a regenerated tooth even when the missing tooth site is large.

Solution

The present invention provides a method for manufacturing a regenerated tooth unit comprising a step of disposing and culturing a regenerated tooth germ inside a spacer in vivo in the body of a mammal, wherein said spacer is configured so as to enable prevention of excessive pressure being applied to said regenerated tooth germ from a tissue inside the body of said mammal, and prevention of said regenerated tooth germ being extended to more than the maximum tolerance; and configured such that the regenerated tooth germ disposed inside is communicatable with the exterior of the spacer. A method for manufacturing a regenerated tooth unit comprising a step of disposing and culturing multiple regenerated tooth germs inside a spacer in vivo in the body of a mammal is also provided.

## Description

### Technical Field

The present invention relates to a method for manufacturing a regenerated tooth unit with controllable size and shape, etc.

### Background Art

The tooth is an organ that has hard tissues of enamel on the outermost layer and dentin on the inner layer thereof, odontoblast which produces dentin further inside, and dental pulp at the center. The tooth may be lost by caries or periodontal disease, and since the presence or absence of teeth has a large influence on appearance or taste of food, as well as maintains health or high quality of life, there is an increased interest towards tooth regeneration technology.
The tooth is a functional unit formed by induction during the developmental process in the fetal stage and constructed by multiple cell strains. A tooth is thus not developed by the stem cell system where a cell strain is developed from a stem cell such as a hematopoietic stem cell or a mesenchymal stem cell in an adult, and it is currently not possible to regenerate a tooth only with implantation of stem cells (stem cell implantation therapy) which is being promoted by regenerative medicine. Regeneration of tooth by identifying a gene specifically expressed in the developmental process of tooth and artificially inducing the tooth germ has also been investigated, but it is not possible to completely induce tooth regeneration only by specification of genes.

Accordingly, a method of obtaining a regenerated tooth inside the mouth by reconstituting a tooth germ with an isolated tooth germ-derived tissue or cell, culturing this to obtain a regenerated tooth unit consisting of a tooth and periodontal tissue, and transplanting the said regenerated tooth unit has been recently investigated.

The present inventors have thus far found that by employing mesenchymal cells and epithelial cells wherein at least either one of these cells is tooth germ-derived cells, and disposing a first cell aggregate consisting substantially of mesenchymal cells and a second cell aggregate consisting substantially of epithelial cells in contact inside a support carrier consisting of e.g. collagen gel, and then culturing the first and second cell aggregates inside the support carrier, a regenerated tooth germ or regenerated tooth unit having effectively induced cell differentiation as well as specific cell disposition and direction can be produced (see e.g. Patent Document 1).

It was also shown that a regenerated tooth germ or regenerated tooth unit having specific cell disposition and direction is similarly obtained when oral epithelial cells or primary cultured cells thereof are utilized as the epithelial cells (see e.g. Patent Document 2), when amnion-derived cells are utilized as the mesenchymal cells (see e.g. Patent Document 3), and when cells differentiation-induced from totipotent stem cells are utilized as the mesenchymal cells (see e.g. Patent Document 4).
It was further shown that the number of regenerated teeth formed can be controlled by adjusting the number of enamel knots comprised in the regenerated tooth germ when producing the regenerated tooth germ (see e.g. Patent Document 5).

Because the size or shape of a tooth varies depending on the site or individual difference, it is important to control the size or shape when producing a regenerated tooth germ or regenerated tooth unit. In particular, when regenerating a tooth compatible to the lost tooth site, the necessary function cannot be exerted unless a tooth having a size and shape equivalent to the lost tooth is produced.
As a method for producing a regenerated tooth having a desired size or shape, there is also proposed a method of seeding a tooth germ cell mixture comprising dental pulp-derived mesenchymal cells that form dental papilla or dentin and epithelial cells that contribute to enamel formation on a scaffold produced by hardening a biodegradable polymer consisting of e.g. polyglycolic acid-polyacetic acid copolymer, transplanting this in the body of an animal to form a tooth. In this method, an attempt to control the shape of the tooth is made by using a scaffold of a given shape. However, since regenerated tooth is derived from a tooth germ consisting of an epithelial cell layer and a mesenchymal cell layer, and the tooth germ grows with epithelial/mesenchymal interaction that occurs overtime between the epithelial and the mesenchymal cell layers, there are opinions that the use of a scaffold is undesirable because sufficient intercellular interaction will not be obtained when a scaffold is employed (see e.g. Non-Patent Document 1). In addition, since the speed of tooth formation is faster than the time for scaffold degradation, it is anticipated that a tooth having involved scaffold may be formed, and the reproducibility of cell disposition or shape of tooth is not necessarily high.

In the meantime, a method of transplanting and culturing a regenerated tooth germ in vivo in the body of an animal is also employed when producing a regenerated tooth unit. When cultured in vivo, the graft easily forms a histologically normal tissue, and cultivation of the tooth and periodontal tissue can also be accelerated, because actions of various cytokine from the animal cell are received and nutrients are supplied from the surrounding. An example of in vivo transplantation widely employed is subrenal capsule transplantation.
However, when the regenerated tooth germ is cultured inside the subrenal capsule as an example of inside the body, the size or shape of the regenerated tooth may be restricted in relation to capsular pressure or transplantation space. Specifically, the regenerated tooth germ is applied pressure from the side, and the tooth occlusal surface becomes vulnerable to having a crushed shape.
Controlling tooth extension is also difficult in in vivo culturing. In particular, the regenerated tooth cannot be transplanted as it is to the jaw bone if it grows to longer than the necessary length.

In the method for culturing a regenerated tooth germ in vivo in the body of an animal, it is also predicted that the development and growth of the periodontal ligament will be insufficient because there is little mechanical stimulation. The periodontal ligament has the function of joining the tooth root and the alveolar bone, the function of alleviating the pressure from the tooth, and the function of rendering the sense of chewiness etc., and is an important element for the regenerated tooth to accomplish equivalent functions as the original tooth.

Patent Document 1
   International Publication No. 2006/129672
Patent Document 2
   Japanese Published Unexamined Patent Application Publication No. 2008-29756
Patent Document 3
   Japanese Published Unexamined Patent Application Publication No. 2008-206500
Patent Document 4
   Japanese Published Unexamined Patent Application Publication No. 2008-200033
Patent Document 5
   Japanese Published Unexamined Patent Application Publication No. 2008-29757
Non-Patent Document 1
   Hu et al. Tissue Engineering Volume 12, Number 8, 2006, 2069-2075

### Summary of the Invention

### Problems to be Solved by the Invention

The object of the present invention is to solve the problem accompanying in vivo culturing of regenerated tooth germ. Specifically, it is an object to provide a method for culturing a regenerated tooth germ so that the shape of the tooth is not restricted by pressure or space even when cultured in vivo and the extension of the tooth can also be controlled, as well as to provide a method which allows sufficient periodontal ligament to be formed around the tooth root. It is also an object to provide a method which allows transplantation of a regenerated tooth even when the missing tooth site is large.

### Means for Solving the Problems

As a result of repeated research to solve the above problems, the present inventors found that when culturing a regenerated tooth germ in vivo in the body of an animal, deformation due to pressure from the tissue of the said body can be prevented by disposing a regenerated tooth germ inside a spacer, and the length of the tooth can also be controlled so that it does not extend to longer than the necessary length. The present inventors also found that when culturing a regenerated tooth germ in vivo in the body of an animal, the periodontal ligament is sufficiently developed around the tooth root by applying mechanical stimulation from the exterior of the said animal. The present inventors further found that when culturing a regenerated tooth germ in vivo in the body of an animal, a regenerated tooth unit comprising multiple regenerated tooth germs or regenerated teeth can be obtained by arranging and culturing individually prepared multiple regenerated tooth germs, and can be transplanted to a large missing tooth site as it is without separation etc.

In other words, the present invention relates to a method for manufacturing a regenerated tooth unit for culturing a regenerated tooth germ in vivo in the body of a mammal, comprising the steps of:
preparing a spacer;
disposing said regenerated tooth germ inside said spacer in vivo in the body of said mammal;
culturing said regenerated tooth germ in vivo in the body of said mammal;
wherein,
said spacer is configured so as to enable prevention of said regenerated tooth germ being extended to more than the maximum tolerance and such that the regenerated tooth germ disposed inside is communicatable with the exterior of the spacer.

One embodiment of the method for manufacturing a regenerated tooth unit of the present invention is characterized in that said mammal is a non-human mammal.

One embodiment of the method for manufacturing a regenerated tooth unit of the present invention is characterized in that said spacer is further configured so as to enable prevention of excessive pressure being applied to said regenerated tooth germ from a tissue inside the body of said mammal.

One embodiment of the method for manufacturing a regenerated tooth unit of the present invention is characterized in that
the step of disposing said regenerated tooth germ inside said spacer in vivo in the body of said mammal is
a step of disposing so that the future occlusal surface site of the regenerated tooth germ is faced in the vicinity of the inner wall of said spacer, and disposing so that the future root apex site extends towards a larger space in a direction opposite to the said inner wall of said spacer.

One embodiment of the method for manufacturing a regenerated tooth unit of the present invention is characterized in that
the step of disposing said regenerated tooth germ inside said spacer in vivo in the body of said mammal is disposing so that said regenerated tooth germ is supported by a support carrier that fills the interior of said spacer.

One embodiment of the method for manufacturing a regenerated tooth unit of the present invention is characterized in that said regenerated tooth germ is cultured and maintained by disposing a first cell assembly composed substantially of mesenchymal cells and a second cell assembly composed substantially of epithelial cells in close contact inside a support carrier, wherein at least either one of mesenchymal and epithelial cells is tooth germ-derived.

Another embodiment of the method for manufacturing a regenerated tooth unit of the present invention is characterized in further comprising a step of applying mechanical stimulation from the exterior of said mammal when culturing said regenerated tooth germ.

Another embodiment of the method for manufacturing a regenerated tooth unit of the present invention is characterized in that said mechanical stimulation is vibration at about 20000 Hz to about 40000 Hz.

One embodiment of the method for manufacturing a regenerated tooth unit of the present invention is characterized in that said spacer has an interior space which enables the development of a regenerated tooth unit having a desired regenerated tooth unit size.

Another embodiment of the method for manufacturing a regenerated tooth unit of the present invention is characterized in that said spacer is rough cylindrical, in which the height of the said cylinder is greater than the thickness of the desired regenerated tooth unit, and the inner diameter of the said cylinder is the approximate maximum tolerance of the length of the regenerated tooth unit.

Another embodiment of the method for manufacturing a regenerated tooth unit of the present invention is characterized in that said spacer is circular cylinder-shaped.

Another embodiment of the method for manufacturing a regenerated tooth unit of the present invention is characterized in that said regenerated tooth germ comprises individually prepared multiple regenerated tooth germs.

Another embodiment of the method for manufacturing a regenerated tooth unit of the present invention is characterized in that said regenerated tooth germ comprises individually prepared multiple regenerated tooth germs, and the individually prepared multiple regenerated tooth germs are disposed adjacent to each other inside said spacer.

Another embodiment of the method for manufacturing a regenerated tooth unit of the present invention is characterized in that said regenerated tooth germ comprises individually prepared multiple regenerated tooth germs, and the individually prepared multiple regenerated tooth germs are disposed in rough parallel alignment in the axial direction that connects the center of the future occlusal surface site of each regenerated tooth germ and the center of the future root apex site inside said spacer.

According to yet another aspect of the present invention, the present invention also relates to a regenerated tooth germ composition for manufacturing a regenerated tooth unit by disposing inside a spacer and culturing in vivo in the body of a mammal, characterized in that said spacer is configured so as to enable prevention of said regenerated tooth germ being extended to more than the maximum tolerance and such that the regenerated tooth germ disposed inside is communicatable with the exterior of the spacer.

Another embodiment of the regenerated tooth germ composition of the present invention is characterized in that said regenerated tooth germ comprises individually prepared multiple regenerated tooth germs.

According to yet another aspect of the present invention, the present invention also relates to a spacer for manufacturing a regenerated tooth unit by placing and culturing a regenerated tooth germ in vivo in the body of a mammal, characterized in that the spacer is configured so as to enable prevention of said regenerated tooth germ being extended to more than the maximum tolerance and such that the regenerated tooth germ disposed inside is communicatable with the exterior of the spacer.

Another embodiment of the spacer of the present invention is characterized in that the regenerated tooth germ disposed inside said spacer comprises individually prepared multiple regenerated tooth germs.

According to yet another aspect of the present invention, the present invention also relates to a method for manufacturing a regenerated tooth unit comprising a step of culturing regenerated tooth germ in vivo in the body of a mammal, wherein
mechanical stimulation is applied from the exterior of said mammal when culturing said regenerated tooth germ.

In addition, another embodiment of the method for manufacturing a regenerated tooth unit of the present invention is characterized in that said mechanical stimulation is vibration at about 20000 Hz to about 40000 Hz.

In addition, another embodiment of the method for manufacturing a regenerated tooth unit of the present invention is characterized in that said regenerated tooth germ is cultured and maintained by disposing a first cell assembly composed substantially of mesenchymal cells and a second cell assembly composed substantially of epithelial cells in close contact inside a support carrier, wherein at least either one of mesenchymal and epithelial cells is tooth germ-derived.

In addition, in another embodiment of the method for manufacturing a regenerated tooth unit of the present invention is characterized in that said regenerated tooth germ comprises individually prepared multiple regenerated tooth germs.

According to yet another aspect of the present invention, the present invention also relates to a method for repairing a missing tooth of an animal, comprising
a step of transplanting a regenerated tooth unit manufactured with the above method for manufacturing a regenerated tooth unit to said missing part.

One embodiment of the method for repairing a missing tooth of an animal of the present invention is characterized in that said animal is a non-human mammal.

One embodiment of the method for repairing a missing tooth of an animal of the present invention is characterized in that said regenerated tooth unit comprises multiple teeth in which the tooth crown-root direction is aligned in rough parallel.

According to yet another aspect of the present invention, the present invention also relates to a regenerated tooth unit manufactured with the above method for manufacturing a regenerated tooth unit.

According to yet another aspect of the present invention, the present invention also relates to a regenerated tooth unit manufactured with the above method for manufacturing a regenerated tooth unit for transplanting to the missing part in order to repair the missing tooth in the mouth of an animal.

Another embodiment of the regenerated tooth unit of the present invention is characterized in that said regenerated tooth unit comprises multiple teeth in which the tooth crown-root direction is aligned in rough parallel.

Another embodiment of the regenerated tooth unit of the present invention is characterized in that said regenerated tooth unit comprises multiple teeth in which the tooth crown-root direction is aligned in rough parallel, and the periodontal tissue is monolithically formed.

### Advantages of the Invention

According to the present invention, a tooth can be grown in the natural shape without excessive pressure being applied from a tissue etc. of the said animal even when the regenerated tooth germ is cultured in vivo in the body of an animal. It is also possible to prevent the tooth from extending to more than necessary.
Further, according to the present invention, a regenerated tooth having sufficient periodontal ligament tissue can be obtained even when the regenerated tooth germ is cultured in vivo in the body of an animal.
Further, according to one aspect of the present invention, a regenerated tooth unit comprising multiple regenerated teeth can be transplanted at once to a large missing bone site without transplanting individual regenerated tooth units one at a time. Further, according to one aspect of the present invention, since the alveolar bone of multiple regenerated teeth is monolithically formed, transplantation as a monolith rigidly supported by a monolithic alveolar bone is possible without the directions of multiple regenerated teeth being varied at the time of or after transplantation.

### Brief Description of the Drawings

Figure 1 is the photograph showing the appearance of performing in vivo culturing of regenerated tooth germ inside a spacer disposed in the mouse subrenal capsule according to the method for manufacturing the regenerated tooth unit of the present invention;
Figure 2 is the micro CT image of a regenerated tooth unit produced with a spacer;
Figure 3 is the micro CT image of a regenerated tooth unit produced without a spacer;
Figure 4 shows the result of the major/minor axis ratios of a regenerated tooth unit produced with a spacer (controlled), a regenerated tooth unit produced without a spacer (uncontrolled), and the tooth crown portion of a natural tooth as measured and calculated on CT images;
Figure 5 shows the result of the lengths of a regenerated tooth unit produced with a spacer (controlled) and a regenerated tooth unit produced without a spacer (uncontrolled) as measured on CT images;
Figure 6 is the HE staining image of a regenerated tooth unit produced with application of mechanical stimulation during in vivo culturing of regenerated tooth germ and a regenerated tooth unit produced without applying stimulation;
Figure 7 is the result of the widths of the periodontal ligament of a regenerated tooth unit produced with application of mechanical stimulation during in vivo culturing of regenerated tooth germ and a regenerated tooth unit produced without applying stimulation as measured on CT images;
Figure 8 shows the appearance of jaw bone transplantation of a regenerated tooth unit manufactured with the method of the present invention;
Figure 9 is the CT image on Day 0, Day 14, and Day 30 after jaw bone transplantation of a regenerated tooth unit manufactured with the method of the present invention;
Figure 10 is the HE staining image on Day 30 after jaw bone transplantation of a regenerated tooth unit manufactured with the method of the present invention;
Figure 11 is the plane view and side view schematically showing a regenerated tooth germ in the state of being disposed inside a spacer;
Figure 12 is the schematic diagram of multiple regenerated tooth germs in the state of being disposed inside a spacer;
Figure 13 is the stereophotograph of multiple regenerated tooth germs in the state of being disposed inside a spacer;
Figure 14 is the stereophotograph of a multiple-teeth regenerated tooth unit at 60 days after subrenal capsule transplantation;
Figure 15 is the CT image of a multiple-teeth regenerated tooth unit at 60 days after subrenal capsule transplantation. (a) shows the CT appearance image, (b) left is the CT cross-sectional image in the dentition direction, and (b) right is the CT cross-sectional image in the buccolingual direction; and
Figure 16 is the CT image on Day 1 after transplantation of multiple-teeth regenerated tooth unit into the mouth.

### Description of Embodiments

The first aspect of the method for manufacturing a tooth according to the present invention is a method for manufacturing a regenerated tooth unit comprising a step of disposing and culturing a regenerated tooth germ inside a spacer in vivo in the body of a mammal, characterized in that the spacer is configured so as to enable prevention of excessive pressure being applied to the regenerated tooth germ from a tissue inside the body of a non-human mammal, and prevention of the regenerated tooth germ being extended to more than the maximum tolerance; and configured such that the regenerated tooth germ disposed inside is communicatable with the exterior of the spacer. "Communicatable" herein means that substances such as nutritive matter or cytokine can permeate between the interior and the exterior of a spacer through the spacer, in other words a substance supply route exists.

A "tooth" herein refers to a tissue comprising continuous layers of dentin on the inside and enamel on the outside, and means a tissue having a tooth crown or tooth root provided with direction. The direction of a tooth can be specified by the positioning of the tooth crown or tooth root. The tooth crown or tooth root can be visually identified based on for example the shape or tissue staining. The tooth crown refers to the portion having the layered structure of enamel and dentin, and no enamel layer exists in the tooth root.

Dentin and enamel can be easily morphologically specified by those skilled in the art by e.g. tissue staining. Enamel can also be specified by the presence of ameloblast, and the presence of ameloblast can be verified by the presence or absence of amelogenin. On the other hand, dentin can be specified by the presence of odontoblast, and the presence of odontoblast can be verified by the presence or absence of dentin sialoprotein. The verification of amelogenin and dentin sialoprotein can be easily carried out by methods known in the art, examples of which include in situ hybridization and antibody staining.

A "tooth germ" herein is an early germ of a tooth that is defined to be a tooth in the future, and refers to those in the stages from the bud stage to the bell stage generally employed in the developmental stage of the tooth, in particular a tissue in which accumulation of dentin and enamel which are the characteristics as the hard tissues of the tooth is not observed. On the other hand, a "tooth bud" herein refers to a tissue at a later stage then the "tooth germ," and refers to a tissue after the stage where accumulation of dentin and enamel which are the characteristics of the hard tissues of the tooth has begun and before the stage where the tooth erupts from the gingiva and generally expresses the functions as a tooth.
A "regenerated tooth germ" herein refers to a tooth germ artificially formed by a cell culture technology.

A "regenerated tooth unit" herein refers to a unit consisting of a tooth and the periodontal tissue supporting the tooth, which is obtained as a result of culturing a regenerated tooth germ and differentiating various cells comprised in the regenerated tooth germ.
The configuration of the tooth portion in the regenerated tooth unit is in the stage between a tooth bud as described above and a tooth. In addition, the periodontal tissue comprises the periodontal ligament and the alveolar bone.
In other words, a regenerated tooth unit is obtained when a regenerated tooth germ is cultured in vivo in the body of a mammal, and a regenerated tooth is obtained by transplanting the regenerated tooth unit in the mouth and allowing integration, or eruption and growth.

A spacer herein means an appliance disposed such that it covers the regenerated tooth germ, so that the regenerated tooth germ does not extend to more than necessary when culturing the regenerated tooth germ in vivo in the body of a mammal. The spacer can also play a role in keeping the regenerated tooth germ from being applied capsular pressure or excessive pressure from a tissue inside the body, for example when culturing inside the body such as the subrenal capsule. The shape of the spacer may be any shape as long as these purposes are achieved.

In other words, the shape of the spacer may be any that can secure the interior space which will allow the development of a regenerated tooth unit having a desired regenerated tooth unit size (mainly length and thickness). The length of the regenerated tooth unit refers to the length from the future occlusal surface site to the future root apex site in the tooth portion of the regenerated tooth unit. It will mean the distance to its tip (the site farthest from the future occlusal surface site) when the periodontal tissue is formed around the future root apex site. The approximate maximum tolerance of the length of the regenerated tooth unit means the extent of the maximum value tolerated as the length of the regenerated tooth unit obtained after culturing the regenerated tooth germ in vivo in the body of a mammal. The said approximate maximum tolerance can be arbitrarily determined by those skilled in the art according to the subject or site to which the regenerated tooth germ is later transplanted. The thickness of the regenerated tooth unit generally refers to the thickness of the tooth crown portion of the regenerated tooth unit, but it may refer to the thickness of the portion having the periodontal tissue when the periodontal tissue is formed around the future root apex site. As with the teeth of human beings, the cross-section of tooth crown portion is ordinarily neither perfect square nor rectangle, but is rather defined by the major and minor axes.

From conventional knowledge, since the direction of extension of the regenerated tooth germ is controllable to a considerable extent, the direction of the regenerated tooth germ composition per se when disposing the regenerated tooth germ composition inside the spacer or its relative position inside the spacer can be arbitrarily adjusted aiming at the desirable extension direction. Accordingly, the extension direction of the regenerated tooth germ composition can be considered in designing the three-dimensional size of the spacer in order to obtain the desired size.
In general, the interior space of the spacer must be larger than the desired regenerated tooth unit. That is to say, the interior space of the spacer has a role in preventing the regenerated tooth unit from extending to more than the maximum tolerance.

A case where the shape of the spacer is a circular cylinder (ring-shaped) with open top and bottom will be described below. Those skilled in the art, however, may easily design a spacer having the desired shape based on the disclosures herein etc.
In case of such a circular cylinder-shape (ring-shape) with open top and bottom, a nutritional supply route from the exterior of the spacer is also sufficiently secured through the opening at the top and bottom, and the formation of a regenerated tooth unit is enabled.
In addition, when employing such a circular cylinder-shaped (ring-shaped) spacer, setting the extension direction of the regenerated tooth germ as the lateral direction of the circular cylinder ring (i.e. the radial direction of the circular cylinder), the longitudinal direction of the circular cylinder (i.e. the height of the circular cylinder) may be set so that the desired thickness of the occlusal surface of the regenerated tooth unit is obtained and the maximum tolerance is not exceeded. In addition, the inner diameter of the ring may be set aiming at the approximate maximum tolerance of the length of the regenerated tooth unit in order to prevent the regenerated tooth unit from extending to more than necessary.
In case of employing a spacer with such a circular cylinder-shape, if the radial direction of the ring is set as the extension direction, when the regenerated tooth germ is disposed so that the longitudinal direction (height direction) will be the width of the tooth crown of the regenerated tooth unit, the height of the cylinder is for example 3-15 mm, preferably 5-12 mm when culturing a human tooth, and 0.4-2.0 mm, preferably 0.6-1.8 mm when culturing a mouse tooth, and its inner diameter (inside diameter) is 10-30 mm, preferably 15-26 mm when culturing a human tooth, and 0.9-2.0 mm, preferably 1.2-1.9 mm when culturing a mouse tooth.

Further, when utilizing a rough circular cylinder-shaped spacer, by disposing the future occlusal surface site of the regenerated tooth germ in great vicinity of the inner wall of the spacer facing the inner wall, and disposing the future root apex site towards the radial direction of the cylinder, the regenerated tooth germ is not applied excessive pressure from a tissue inside the body, the regenerated tooth germ is prevented from extending to more than necessary, and is communicatable with the exterior and substance supply route can be secured.
The occlusal surface of the regenerated tooth germ can grow in a natural shape without being crushed by pressure, the tooth will extend in the diameter direction of the cylinder, and in general, the extension can be stopped at a length shorter than the maximum tolerance before or after reaching the opposite wall.
The regenerated tooth germ can avoid the pressure in the horizontal direction of the cylinder by virtue of the spacer wall, as well as mostly avoid the pressure in the top-bottom direction by virtue of the top and bottom edges of the spacer.

Those skilled in the art can employ a spacer of any desired shape without being limited to a circular cylinder-shape in the manufacturing of a regenerated tooth unit in vivo, in order to achieve the desired purpose defined herein, or in order to achieve other proposes necessary in some instances. Examples include, but are not limited to, those having an elliptic or polygonal base instead of a circular cylinder, or those having a sphere shape.

The spacer of the present invention is configured such that the regenerated tooth germ disposed inside is communicatable with the exterior of the spacer. This is because since the regenerated tooth germ receives a supply of nutrients etc. from a tissue inside the body of a mammal or receives actions of cytokines produced by the animal cell, it is necessary to secure a supply route for these substances to reach the regenerated tooth germ inside the spacer. In order for it to be communicatable, the shape of the spacer, regardless of whether large or small or the number, can form pore(s) sufficient for supplying the substance. For example, it may have a partial large pore such as in the case of a cylinder, or it may have numerous small pores such as in the case of a mesh throughout the spacer. In addition, it may be without a pore as long as the substance can penetrate the material of the spacer.

A mammal herein is not particularly limited as long as it is able to culture a regenerated tooth germ in vivo inside the body, and may be a non-human mammal. Examples include a cow, a horse, a pig, a dog, a cat, and a mouse, preferably a pig and a mouse. It is also particularly preferred that it is the same species as the tooth germ tissue. When transplanting and culturing in an animal that is not the same species as the tooth germ tissue, it is preferred to employ an animal modified to be immunodeficient. In order to allow the development of an organ or tissue of an animal cell to be as normal as possible, the subrenal capsule, the mesenterium, and the subcutaneous etc. are preferred as the favorable in vivo body site for in vivo growth.
The culture period after transplantation varies depending on the size of tooth at the time of transplantation and the size of tooth to be developed, and can generally be between 3 to 400 days. For example, when transplanting in the subrenal capsule, although varied depending on the size of the tooth germ to be transplanted and the size of tooth to be regenerated, about 7 to 60 days is preferred.

The material of the spacer is not particularly limited and it may be any having sufficient rigidity against the pressure from a tissue inside the body, examples of which include gold, silver, iron, copper, aluminum, polyethylene, resin, dental resin, and dental cement.

In addition, in the method for manufacturing a regenerated tooth unit according to the present invention, the step of disposing and culturing a regenerated tooth germ inside a spacer is preferably performed by filling the spacer with a support carrier, and disposing the regenerated tooth germ inside the said support carrier.
The support carrier is not particularly limited as long as it enables cell culture inside, but is preferably e.g. gel, fiber, or solid, and excessive pressure can be further prevented from being applied to the regenerated tooth germ in vivo by employing such support carrier.
Examples of a support carrier employed in the present invention include collagen, agarose gel, carboxymethylcellulose, gelatin, agar, hydrogel, Cellmatrix (product name), Mebiol 1 Gel (product name), Matrigel (product name), elastin, fibrin, laminin, extracellular matrix mixture, polyglycolic acid (PGA), polylactic acid (PLA), and lactic acid/glycolic acid copolymer (PLGA). Among these, collagen, agarose gel, carboxymethylcellulose, gelatin, agar, hydrogel, Cellmatrix, Mebiol Gel, Matrigel, extracellular matrix mixture, elastin, fibrin, and laminin having appropriate hardness or retentivity are preferred.
For example, a liquid support carrier may be employed and hardened after disposing the regenerated tooth germ. For example, after filling the spacer with a collagen solution and disposing the regenerated tooth germ into the collagen solution, the collagen can be gelled by culturing at 37°C in a CO₂ incubator. The regenerated tooth germ can then be disposed in vivo in the body of a mammal along with the spacer and cultured.

In addition, another aspect of the present invention is a method for manufacturing a tooth comprising a step of culturing regenerated tooth germ in vivo in the body of a mammal, characterized in that mechanical stimulation is applied from the exterior of the mammal when culturing the regenerated tooth germ.
The formation of the periodontal ligament in the regenerated tooth unit is promoted by applying mechanical stimulation. Examples of mechanical stimulation include, but are not limited to, vibration, compression, and friction. For example, it is preferred to apply vibration at a near sonic wave frequency (e.g. about 20000 Hz to 40000 Hz).

In the method for manufacturing a regenerated tooth unit employing the spacer according to the present invention stated above, it is also preferred that mechanical stimulation is applied from the exterior of the mammal when culturing the regenerated tooth germ in vivo.

The regenerated tooth germ employed in the method of the present invention will now be described.
The regenerated tooth germ may be those produced by any method, for example, it can be produced by a method comprising the steps of: disposing a first cell assembly composed substantially of mesenchymal cells and a second cell assembly composed substantially of epithelial cells in close contact; and culturing the first and second cell assemblies inside a support carrier. In the present specification, although the cell assembly composed substantially of mesenchymal cells is referred to as the first cell assembly for convenience in order to discriminate the two cell assemblies, it is also possible to refer to the cell assembly composed substantially of epithelial cells as the first cell assembly, and it is not essential which of these is referred to as the first cell assembly.

A "mesenchymal cell" in the present invention means a mesenchymal tissue-derived cell and a cell obtained by culturing this cell, and an "epithelial cell" means an epithelial tissue-derived cell and a cell obtained by culturing this cell.
In addition, a "periodontal tissue" in the present invention refers to the alveolar bone and the periodontal ligament formed mainly on the outer layer of the tooth. The alveolar bone and the periodontal ligament can be easily morphologically specified by those skilled in the art by e.g. tissue staining.

The "step of disposing a first cell assembly composed substantially of mesenchymal cells and a second cell assembly composed substantially of epithelial cells in close contact" is for example described in Patent Documents 1 to 5, the disclosures of each of these references incorporated herein by reference in its entirety.

The above first cell assembly and second cell assembly are each composed substantially of mesenchymal cells only or epithelial cells only. "Composed substantially of mesenchymal cells only" in the present invention means that one cell assembly is in a state that serves the same function as when it is composed of mesenchymal cells only, and comprises as little as possible anything other than cells that become a mesenchymal cell. The same goes in the case of "consisting substantially of epithelial cells only."
A cell assembly here refers to the state of cells in close contact, and may be a tissue or a cell aggregate prepared from loose cells. Employing a tissue has the advantage that a tooth with correct cell disposition or shape will be easily obtained, but the available amount may be limited. Cell aggregates are relatively easy to obtain and preferred because cultured cells can also be employed. According to the method according to the present invention, a regenerated tooth with correct cell disposition or shape can be obtained even when a cell aggregate is employed.

At least either one of mesenchymal and epithelial cells configuring a cell assembly is tooth germ-derived. This allows the cell disposition in vivo to be reproduced, and a tooth having specific structure and direction can be effectively formed. It is more preferred that both mesenchymal and epithelial cells are tooth germ-derived. The said tooth germ is preferably one from the bud stage to cap stage with respect to the blastogenicity and homogenicity of cell differentiation stage.

As mesenchymal cells derived from other than the tooth germ, cells derived from other mesenchymal tissues in the body can also be employed. These are preferably blood cell-free bone marrow cells or mesenchymal cells, further preferably oral mesenchymal cells or bone marrow cells inside the jaw bone, mesenchymal cells derived from cephalic neural crest cells, and mesenchymal progenitor cells that may differentiate into these mesenchymal cells or stem cells thereof etc. An example of employing amnion-derived cells as the mesenchymal cells is described in Patent Document 3, and an example of employing cells differentiation-induced from totipotent stem cells is described in Patent Document 4, the disclosures of which are incorporated herein by reference in its entirety.

As epithelial cells derived from other than the tooth germ, cells derived from other epithelial tissues in the body can also be employed. These are preferably skin or oral mucosa or gingival epithelial cells, further preferably immature epithelial progenitor cells that may differentiate into differentiated, e.g. keratinized or parakeratinized epithelial cells such as skin or mucosa, for example non-keratinized epithelial cells or a stem cells thereof etc. An example of employing oral epithelial cells or primary cultured cells thereof as the epithelial cells is described in Patent Document 2, the disclosure of which is incorporated herein by reference in its entirety.

The tooth germ and other tissues can be collected from the jaw bone etc. of various animals such as mammalian primates (such as humans and monkeys), ungulates (such as pigs, cows, and horses), small mammalian rodents (such as mice, rats, and rabbits), as well as dogs and cats. Conditions ordinarily employed for tissue collection may be applied without change for collecting the tooth germ and tissue, and may be removed in sterile condition and preserved in an appropriate preservation solution. Examples of a human tooth germ include the tooth germ of the third molar, the so-called wisdom tooth, as well as the fetal tooth germ, but it is preferred to employ the tooth germ of the wisdom tooth with respect to utilization of autologous tissue. In case of a mouse, it is preferred to employ the tooth germ of Embryonic Day 10 to 16.

The preparation of mesenchymal and epithelial cells from a tooth germ is performed by first separating the tooth germ isolated from the surrounding tissue into the tooth germ mesenchymal tissue and the tooth germ epithelial tissue according to its shape. In doing so, an enzyme may be employed to facilitate separation. Examples of an enzyme include dispase, collagenase, and trypsin.

The cell aggregate according to the present invention means aggregated cells derived from the mesenchymal tissue or the epithelial tissue, and can be prepared by aggregating cells obtained by dispersing the mesenchymal tissue or epithelial tissue into bits, or cells obtained by primary or passage culturing of the said cells.

An enzyme such as dispase, collagenase, and trypsin may be employed to disperse cells. When performing primary or passage culturing of dispersed cells before preparing the cell aggregate in order to obtain a sufficient number of cells, the medium employed for culturing that can be employed is a medium generally employed for animal cell culturing such as Dulbecco's Modified Eagle medium (DMEM). A serum for promoting cell proliferation may be added, or a serum alternative, for example a cell growth factor such as FGF, EGF, and PDGF or a known serum component such as transferrin may be added. The concentration when adding a serum can be arbitrarily altered depending the culture state at the time, but is ordinarily around 10 %. For cell culturing, ordinary culture conditions such as culturing in an incubator at a temperature of about 37°C and 5 % CO₂ concentration are applied. An antibiotic such as streptomycin may also be added as appropriate.

In order to aggregate cells, for example, the cell suspension may be centrifugated. It is preferred that the cell aggregates of each of mesenchymal and epithelial cells are kept at a high density state in order to ensure cellular interaction when the two are in close contact. High density state refers to an extent equivalent to the density of constituting a tissue, for example, 5 x 10⁷ cells/ml to 1 x 10⁹ cells/ml, preferably 1 x 10⁸ cells/ml to 1 x 10⁹ cells/ml, and most preferably 2 x 10⁸ cells/ml to 8 x 10⁸ cells/ml. The method for making the cell aggregate high density is not particularly limited, for example it can be performed by a method of aggregating and precipitating the cells by centrifugation. Centrifugation is preferred because it easily enables high density without impairing cellular activity. Centrifugation may be performed at a rotational speed that renders a centrifugal force of 300 x g to 1200 x g, preferably 500 x g to 1000 x g for 3 to 10 minutes. There is a tendency that cell density is unable to be sufficiently increased at centrifugation lower than 300 x g. On the other hand, cells may be damaged at centrifugation higher than 1200 x g.

Ordinarily, when preparing a high density cell aggregate by centrifugal separation, a cellular suspension is prepared in a container such as a tube employed for centrifugal separation of cells before performing centrifugal separation, and the supernatant may be removed as much as possible, leaving the cells as the precipitate. Here, components other than the cells of interest (e.g. culture medium and buffer) are preferably at an amount less than or equal to the cell volume, and most preferably no component other than the cells of interest is comprised. If such high density cell assembly is put into close contact inside a support carrier by the method described below, cells come into tight contact and intercellular interaction is effectively exerted.

A support carrier same as the support carrier disposed inside the spacer as described above can be used. The support carrier employed for the purpose of culturing the first and second cell assemblies is preferably equipped with retentivity that enables retaining the cell assemblies in close contact without the cells being dispersed. In close contact means the state where the high density cell assemblies of mesenchymal and epithelial cells described above also retains a similar extent of density near the contact surface of the mesenchymal and the epithelial cells. The support carrier that enables retention of close contact, in case of collagen for example, is used at a concentration of 2 mg/ml to 3 mg/ml final concentration, i.e. a concentration that gives 120 g to 250 g jelly strength by the method in compliance to JIS-K6503-1996 (measured as the load necessary to push down 4 mm with a 12.7 mm diameter plunger) to provide an appropriate hardness. Other types of support carriers are also preferably employed as the support carrier of the present invention if it has a similar strength by a similar evaluation method. A support carrier having a hardness corresponding to the target jelly strength may also be obtained by mixing one or more types of support carriers.

The method of disposing the first and second cell assemblies into the support carrier is not particularly limited, and when the cell assembly is a cell aggregate, for example, the precipitate obtained with the centrifugal separation described above can be inserted into the support carrier with a microsyringe etc. and disposed. When the cell assembly is a tissue, it can be disposed at any position inside the support carrier by employing the tip of a needle of a syringe etc.

The method for disposing the first and second cell assemblies in close contact with the support carrier in the present invention is not particularly limited, for example, the two can be put into close contact by disposing one cell assembly inside the support carrier, and then disposing the other cell assembly so as to push onto it. More specifically, by arbitrarily altering the position of the above tip of the needle of the syringe inside the support carrier, one cell assembly can be pushed onto the other cell assembly. When employing an epithelial or a mesenchymal tissue as the cell assembly, it is preferred to dispose the surface of the said tissue that had contacted the mesenchymal or the epithelial tissue in the original tooth germ in contact with the other cell assembly.
It is also preferred to comprise a step of hardening the support carrier after disposing. This enables the cell to further aggregate to a state of higher density. For example, in the case of collagen gel, it can be solidified by letting it stand under culture temperature for several minutes to several tens of minutes. In this case, less components other than the cell there is in the cell assembly, a state of higher density can be realized.

The "step of culturing the first and second cell assemblies inside a support carrier" in the present invention is for example described in Patent Documents 1 to 4 and Japanese Published Unexamined Patent Application Publication No. 2008-29757, the disclosures of each of these references incorporated herein by reference in its entirety.

The culture period varies depending on for example the number of cells disposed inside the support carrier, the state of the cell assembly, culture conditions, and animal species, as can be arbitrarily selected by those skilled in the art. It is preferred to culture for at least 1 day, more preferably for 3 days or more to allow eruption as a functional tooth when transplanted in the mouth.
By prolonging the culture period, the formation of reconstituted tooth germ such as the accumulation of dentin and enamel, the formation of tooth crown, and the formation of tooth root can be further progressed. In order to obtain the desired state, the culturing may be e.g. for 6 days or more, 30 days or more, 50 days or more, 100 days or more, or 300 days or more, and the medium or culture conditions can also be altered during culture.

The step of culturing inside the support carrier may be culturing the support carrier that encases the first and second cell assemblies alone, or may be culturing in the presence of other animal cells etc.
When the support carrier is cultured alone, the culture conditions can be those employed in general animal cell culturing. A mammal-derived serum may be added to the culture, or various cell factors known to be effective for proliferation or differentiation of these cells may also be added. Examples of these cell factors can include FGF and BMP.

With respect to gas exchange or nutritional supply of the cell assembly, and with respect to no contact or contamination with other animal cells and enabling all steps to be performed in vitro, it is preferred to make culturing inside the support carrier an organ-culturing. In organ-culturing, in general, a porous membrane is floated on a medium suited for animal cell proliferation, and a support carrier that encases the first and second cell assemblies is mounted on the membrane and cultured. The porous membrane employed here is preferably that having numerous pores of about 0.3 to 5 µm, examples of which can include Cell Culture Insert (product name) or Isopore filter (product name).

In the method for manufacturing a tooth according to the present invention, before disposing and culturing the regenerated tooth germ in vivo in the body of a mammal, it is also preferred to preculture the regenerated tooth germ ex vivo. Intercellular attachment and intercellular interaction can be made more rigid by preculturing, and as a result, the entire growth period can be shortened.

The tooth obtained by the method according to the present invention has a cell disposition (structure) specific to tooth which is dentin on the inside and enamel on the outside, preferably also comprises a direction where the tip of the tooth (tooth crown) and the tooth root are in correct positions, and sufficiently serves the functions as a tooth. Accordingly, it can be widely utilized as an alternative to tooth. It can also be employed as a research tool useful in the research to elucidate the developmental process of tooth.

Further, by prolonging the culture period, the periodontal tissue such as the alveolar bone or the periodontal ligament that supports and fixes the tooth on the jaw bone can also be formed in addition to teeth per se. This can further enhance the practicality of the tooth after transplantation. It is also possible to separate and utilize only the periodontal tissue.

### [Method for Repairing Missing Tooth Part]

The method for repairing the missing tooth part in the mouth according to the present invention comprises a step of transplanting a regenerated tooth unit manufactured with the method for manufacturing a regenerated tooth unit according to the present invention to the missing part. According to the present method, it is possible to manufacture and transplant a regenerated tooth unit compatible to the size and shape of the missing part.
According to the method for manufacturing a regenerated tooth unit according to the present invention, when culturing a regenerated tooth germ in vivo, a tooth having a shape compatible to the missing part is easily manufactured because the tooth can be grown to a natural shape without being applied pressure in vivo and crushed.
In addition, it is desired to produce a regenerated tooth having the same length as the missing tooth for transplanting to the missing part, and according to the method for manufacturing a tooth according to the present invention, the maximum value of the length of the tooth can be controlled. When the length of the tooth exceeds the maximum tolerance, the tooth cannot be transplanted as it is, but if it is shorter than the maximum tolerance, it is possible to allow it to grow after transplantation to obtain a regenerated tooth of a desired length.

In the method for repairing the missing tooth part in the mouth according to the present invention, those in any tooth developmental stage may be transplanted as the regenerated tooth unit. It is preferred to dispose so that the tooth crown is on the interior side of the mouth if the formation of tooth crown is already recognized. If it is at a stage where the formation of tooth crown is not recognized, it is preferred to dispose so that the epithelial cell layer corresponding to the tooth crown portion or the epithelial cell layer of the regenerated tooth germ is on the interior side of the mouth. It is also preferred to dispose the opening of the epithelial/mesenchymal cell layer of the regenerated tooth germ to be opposite to the interior side of the mouth. This enables the tip part of the tooth (tooth crown) to be on the interior side of the mouth to have a direction similar to the surrounding teeth.

A missing part means a portion established in the gingiva by e.g. tooth extraction, and there is no particular restriction to the shape. The missing location and the intended type of tooth are not particularly limited as long as the regenerated tooth germ or tooth can be embedded.
The missing part is ordinarily located in the jaw bone or the oral alveolar bone. Moreover, if the loss of tooth is accompanied by reduction in the alveolar bone mass, an attempt to promote regeneration of the periodontal tissue may be made to the missing site by a well-known method clinically employed for embedding an implant such as the GTR method (guided tissue regeneration: tissue regeneration induction method). After disposing the tooth germ or tooth to the hole portion, it is preferred to carry out suturing etc. according to an ordinary treatment.

In the method for repairing the missing tooth part in the mouth according to the present invention, it is preferred that the transplantation subject is the same species as the animal from which the tooth germ employed for manufacturing the tooth was harvested, further preferably the same individual as the individual from which the tooth germ was harvested. Examples of an animal include a mammal including a human, a cow, a horse, a pig, a dog, a cat, and a mouse etc. It is also preferably a non-human mammal.

As another embodiment of the present invention, multiple regenerated tooth germs can be employed as the regenerated tooth germ. It is preferred that the multiple regenerated tooth germs are regenerated tooth germs where each regenerated tooth germ is individually prepared. An individually prepared regenerated tooth germ refers to a regenerated tooth germ prepared to form a single tooth in the future. The method for producing a regenerated tooth germ is as described above. The individually prepared regenerated tooth germ, as described for example in Patent Document 1, can be separated after producing a regenerated tooth germ comprising multiple tooth germs. However, more preferably in general, as described for example in Patent Document 5, it can be produced to form a single tooth in the future by adjusting the number of enamel knots comprised in the regenerated tooth germ. Said individually prepared regenerated tooth germ can also be produced by arbitrarily adjusting its size depending on the size of the missing part or the size of tooth to be regenerated. For example, when disposing said mesenchymal cell assembly and said epithelial cell assembly in contact in the production of a regenerated tooth germ, each cell assembly can be formed in a rough cylindrical shape, and in such a case, a tooth having the desired length in one direction can be formed by controlling the contact length of the axial direction of the cylinder. For example, a regenerated tooth having the said desired length can be produced by making the contact length of said mesenchymal cell assembly and said epithelial cell assembly to be ± 25 % of the width of the tooth crown of the desired regenerated tooth. The length etc. of the cell assembly can be adjusted for production so that it has the size and shape of the desired regenerated tooth as a result of culturing. A regenerated tooth unit comprising multiple regenerated teeth is produced by disposing in sequence and culturing the individually prepared regenerated tooth germ as above. It is preferred to align the axial direction that connects the center of the future occlusal surface site and the center of the future root apex site of the regenerated tooth germs in rough parallel for disposal. The axial direction that connects the center of the future occlusal surface site and the center of the future root apex site roughly matches the axial direction that connects the tooth crown and the tooth root of the regenerated tooth, and this direction may be referred to herein as the "tooth crown-root direction". By aligning this axial direction in rough parallel for disposing, a regenerated tooth unit comprising multiple teeth with the tooth crown-root direction aligned similarly to innate teeth can be obtained. When disposing multiple regenerated tooth germs, it is also preferred to dispose then on one plane. By disposing on one plane, a regenerated tooth unit having ordered dentition can be obtained. When it is desired to have a state where the dentition is aligned along the curve of the gum, disposition can also be on the curved surface along the curve of the gum instead of a plane. When disposing multiple regenerated tooth germs, the number of regenerated teeth or regenerated tooth germs is not particularly limited, and can be selected according to the shape and size of the missing tooth site or the desired number of regenerated teeth. Preferably from 2 to 6, and more preferably from 2 to 4 can be disposed.

When disposing multiple regenerated tooth germs in sequence, it is preferred to dispose the regenerated tooth germs adjacent to each other. Adjacent disposition refers to disposing the regenerated tooth germs in contact or almost in contact with each other. When regenerated tooth germs are disposed adjacent to each other, a regenerated tooth unit with little or no gap between teeth in the dentition direction can be obtained. In addition, when multiple regenerated tooth germs are adjacently disposed and cultured, a regenerated tooth unit having a monolithic shape with the periodontal tissues attached to each other can be obtained with a longer culture period. Accordingly, a regenerated tooth unit with monolithically formed periodontal tissue can be transplanted into the missing tooth site as it is without separating each regenerated tooth. The monolithically formed regenerated tooth unit comprising multiple regenerated teeth as above allows a simpler transplantation compared to when transplanting individual regenerated tooth units one at a time, and individual teeth can be rigidly supported by the monolithic periodontal tissue. In addition, the monolithically formed regenerated tooth unit comprising multiple regenerated teeth can be transplanted with less concern for the dentition direction being disordered at the time of or after transplantation compared to when transplanting individual regenerated tooth units one at a time.

As another embodiment of the present invention, said multiple regenerated tooth germs can also be disposed inside a spacer and cultured as described above. The spacer, similarly to the above, preferably has an appropriate shape in accordance to the number of regenerated tooth germs to be arranged. When arranging multiple regenerated tooth germs, it is preferred to have a shape so that the total of the width of the tooth crown in the dentition direction of the multiple tooth germs or the length of the periodontal tissue in the dentition direction is obtained. Accordingly, when employing a cylindrical spacer, it is preferred to employ a cylinder with a shape close to an ellipse or a rectangle as the base. When producing a regenerated tooth unit comprising multiple regenerated teeth, by employing a spacer, there is an advantage that it can be inserted inside the body for in vivo culturing while maintaining the aligned state of regenerated tooth germs, i.e. maintaining the direction of the regenerated tooth germs or the interval between the regenerated tooth germs as well as the plane on which the regenerated tooth germs are arranged.

The terms used herein are for the purpose of describing particular embodiments and do not intend to limit the invention.
In addition, the term "comprising" as used herein, unless the context clearly indicates to be understood otherwise, intends the presence of the described items (such as components, steps, elements, and numbers), and does not exclude the presence of other items (such as components, steps, elements, and numbers).
Unless otherwise defined, all terms used herein (including technical and scientific terms) have the same meaning as that broadly recognized by those skilled in the art of the technology to which the present invention belongs. The terms used herein, unless explicitly defined otherwise, should be construed as having meanings consistent with the meanings herein and in related technical fields, and are not to be construed as idealized or excessively formal meanings.
The embodiments of the present invention may be described referring to schematic diagrams. In such a case, they may be exaggerated in presentation in order to allow clear description.
Terms such as first and second are employed to express various elements, but it should be recognized that these elements are not to be limited by these terms. These terms are employed solely for the purpose of discriminating one element from another, and for example, it is possible to describe a first element as a second element, and similarly, to describe a second element as a first element without departing from the scope of the present invention.

### Examples

The present invention will now be described in further detail referring to Examples. However, the present invention can be embodied by various aspects, and shall not be construed as being limited to the Examples described herein.

### <Example 1: In Vivo Culture of Regenerated Tooth Germ with Spacer>

In vivo culturing of regenerated tooth germ with a spacer was performed with the purpose of avoiding the influence of renal 1 capsular pressure in subrenal capsule transplantation.

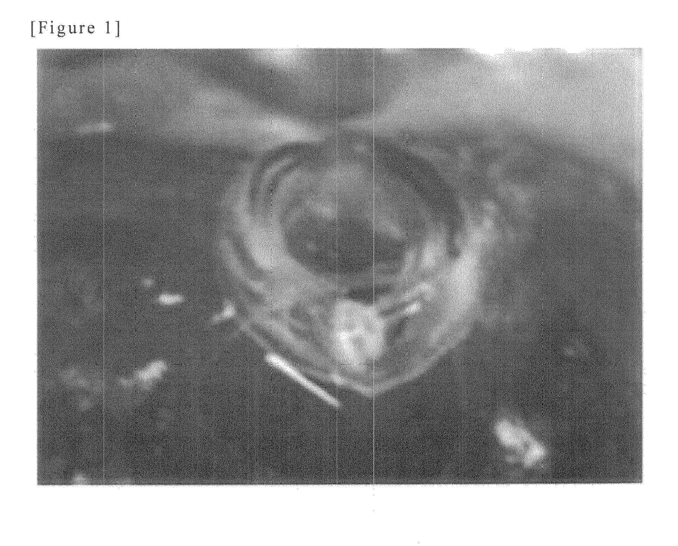

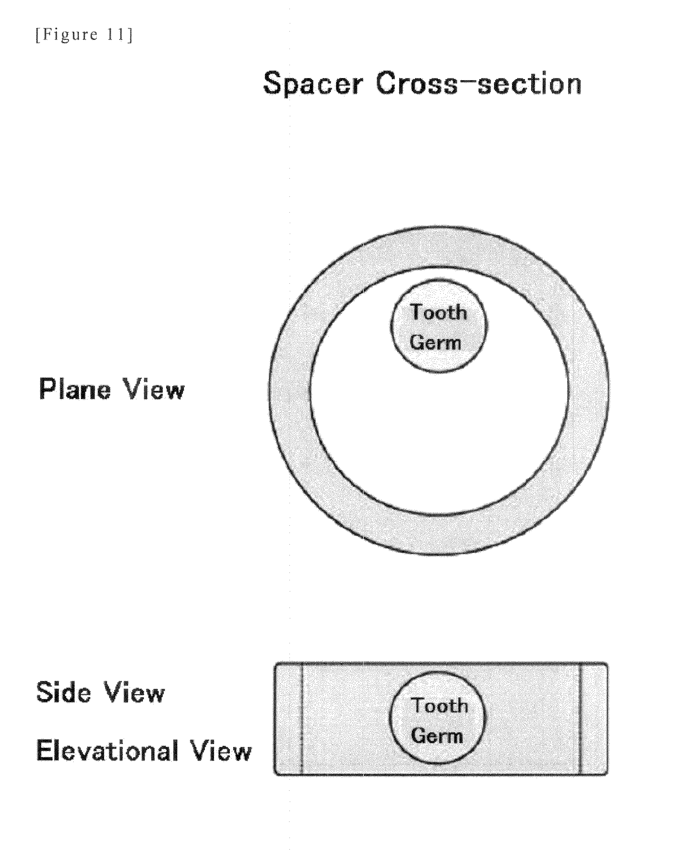

A micropipette chip (HRI-110 NEW, Molecular Bio Products, San Diego, CA, USA) was cut in a ring-shape (cylinder-shape) so that the inner diameter (inside diameter) was 1.3 mm and the height was 1.3 mm to fabricate a spacer, and filled the interior with a collagen solution and used. The regenerated tooth germ was disposed so that the spacer wall surface was in proximity to the tooth crown side in order to promote extension in the tooth root direction, and cultured in a CO₂ incubator (SANYO Electric Co.,Ltd, Osaka, Japan) at 37°C to harden the collagen gel. Next, the spacer comprising a regenerated tooth germ was transplanted to the bilateral subrenal capsule of a 7 weeks-old C57BL/6 mouse. Transplantation to the subrenal capsule was performed by making a 2 to 3 mm incision in the renal capsule, detaching the renal capsule and the renal parenchymal, and inserting between them the spacer comprising a regenerated tooth germ. As a comparative example, a regenerated tooth germ organ-cultured for 7 days was transplanted to the subrenal capsule without a spacer. The photograph at the time of transplantation is shown in Figure 1. The schematic diagram of the positional relationship between the spacer and the regenerated tooth germ at the time of transplantation is also shown in Figure 11.

Micro CT images were taken 3 weeks after transplantation, and the image data were analyzed with an integrated image processing software. The maximum diameter in the tooth crown portion was defined as the major axis and the maximum diameter orthogonal to the major axis was defined as the minor axis, and each length was measured and the ratio thereof calculated.

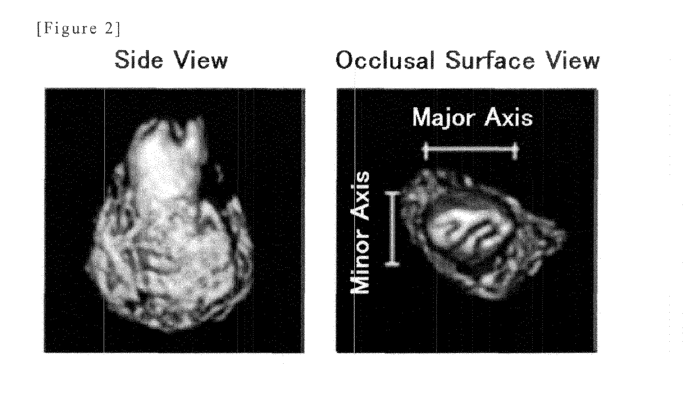

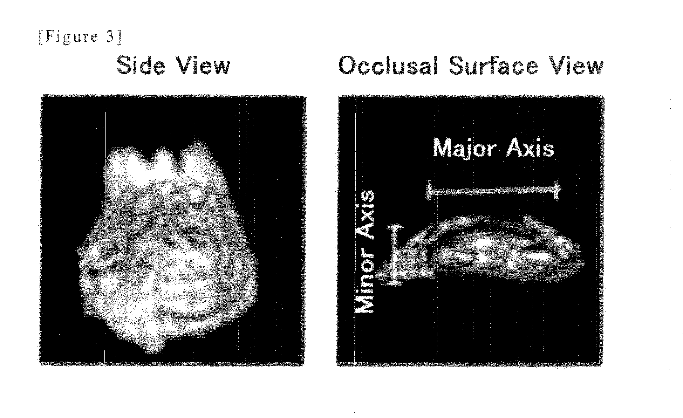

An example of culturing with a spacer is shown in Figure 2, and an example of culturing without is shown in Figure 3. While the regenerated tooth unit cultured with a spacer was in a state close to the normal tooth, the regenerated tooth unit cultured without a spacer had a major axis extremely longer compared to the minor axis, having a flattened shape.

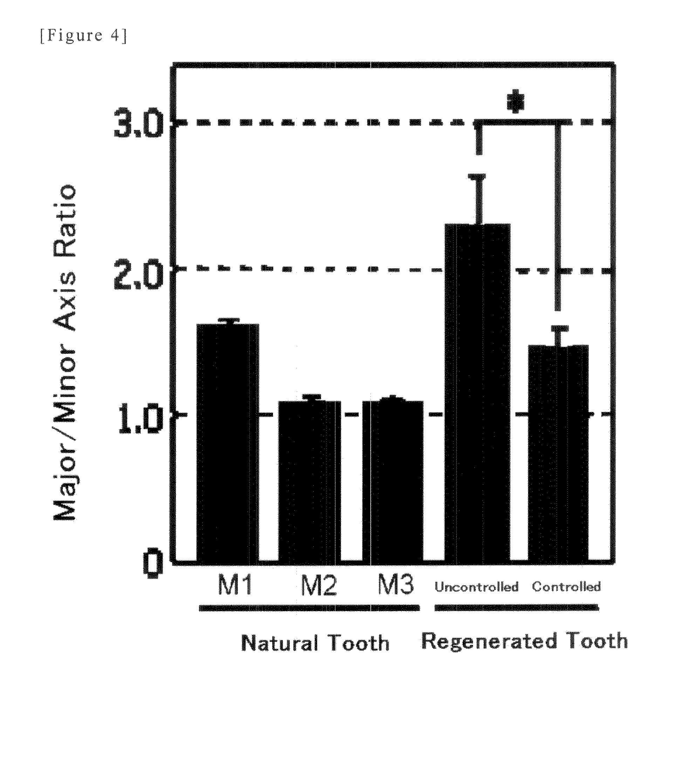

The major and minor axes were measured for each of the 7 cases of the group cultured with a spacer (controlled group) and the group cultured without a spacer (uncontrolled group). The result of calculating the major/minor axis ratio is shown in Figure 4 (right). The major/minor axis ratios of the regenerated tooth unit were 1.46 ± 0.16 mm for the group cultured with a spacer (controlled group: Examples), and 2.30 ± 0.35 mm for the group cultured without a spacer (uncontrolled group: Comparative Examples).

Figure 4 (left) also shows the result of measuring the maximum diameter in the tooth crown portion of the first, second, and third molar of the lower jaw (M1, M2, and M3) of adult mouse defined as the major axis and the maximum diameter orthogonal to the major axis defined as the minor axis, and calculating the major/minor axis ratio. The major/minor axis ratios of M1, M2, and M3 were 1.61 ± 0.05 (n = 5), 1.09 ± 0.04 (n = 5), and 1.12 ± 0.04 (n = 5), respectively.

Consequently, it was confirmed that the shape of the regenerated tooth unit cultured in vivo without a spacer becomes flattened whereas the shape of the regenerated tooth unit cultured in vivo with a spacer will not be flattened and a significant difference exists between the major/minor axis ratios of the two, and that the major/minor axis ratio of the regenerated tooth unit cultured in vivo with a spacer is equivalent to natural teeth (Student's t-test, *p<0.0001).

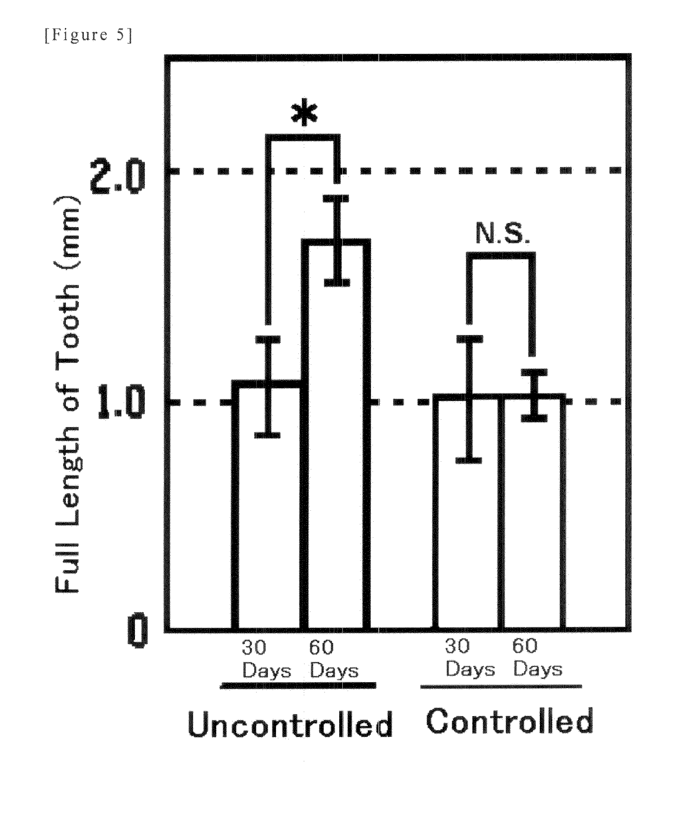

The regenerated tooth unit on Day 30 and Day 60 of subrenal capsule transplantation was also harvested, and micro CT images were taken and analyzed by an integrated image processing software to measure the length. The length from the cusp to the root apex of the tooth was measured on the micro CT image without including the alveolar bone region formed.
The result is shown in Figure 5. In the uncontrolled group without a spacer, the cusp-root apex length was 1.07 ± 0.20 mm (n = 6) on Day 30 as opposed to 1.70 ± 0.26 mm (n = 6) on Day 60, and the length of the tooth had significantly increased. On the other hand, in the controlled group with a spacer, as seen from 1.01 ± 0.19 mm (n = 13) on Day 30 and 1.02 ± 0.11 mm (n = 10) on Day 60, increase in the length of the tooth due to the number of transplantation days was not observed (Student's t-test, *p<0.0001).
Consequently, it became clear that the length of the regenerated tooth can be arbitrarily adjusted by culturing a regenerated tooth germ in vivo with a spacer.

### <Example 2: In Vivo Culture of Regenerated Tooth Germ with Mechanical Stimulation>

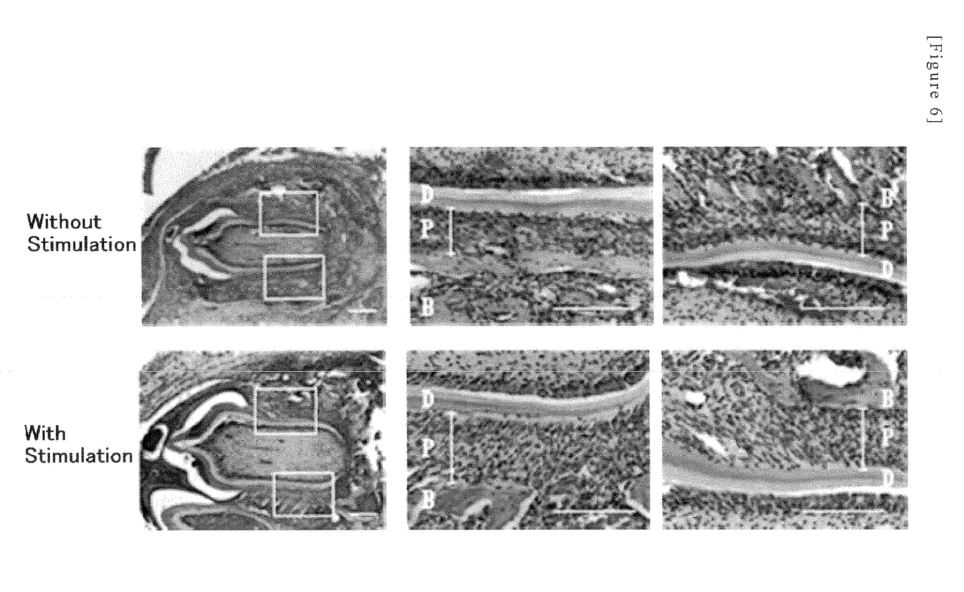

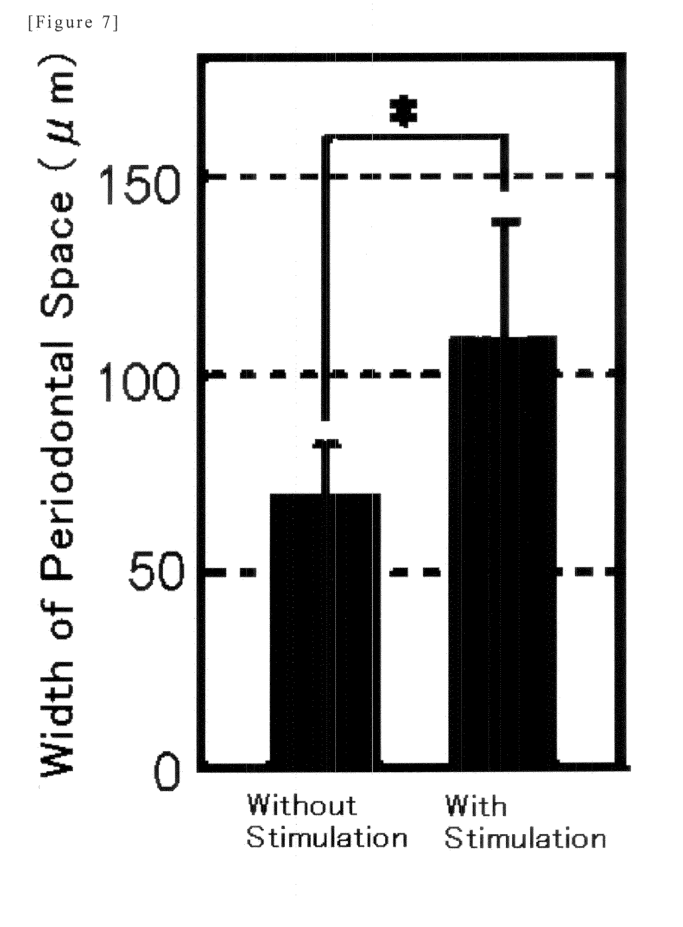

In accordance to the method of Example 1, a regenerated tooth germ organ-cultured with a spacer was transplanted in the subrenal capsule along with the spacer. As mechanical stimulation, a sonic wave electric toothbrush (frequency 31,000 Hz: Doltz™, Panasonic) was pressed onto the mouse dorsal skin at a pressure of 3.9 x 10⁴ Pa (5 minutes/day) from Day 7 after subrenal capsule transplantation. No mechanical stimulation was given to the control group, and these were harvested after 30 days of transplantation period as comparison subjects.
Micro CT images were taken for the regenerated tooth unit harvested with a method similar to Example 1, and the width of the periodontal space (sometimes simply referred to as "the width of the periodontal ligament") was measured using a three-dimensional image analysis software. Specifically, the distance between the tooth root surface of the regenerated tooth unit and the alveolar bone formed on the three-dimensional image was set as the width of the periodontal space, and six points in each of the cross-sections on the major/minor axis were measured. The regenerated tooth was then fixed in 4 % paraformaldehyde (Paraformaldehyde: PFA), decalcified in 10 % ethylenediaminetetraacetic acid (EDTA), and embedded in paraffin. Continuous sections 8 micrometers thick were then created, hematoxylin-eosin (HE) stained, and histological evaluation was performed.
The result of histological evaluation is shown in Figure 6. In the figures, B shows the alveolar bone, D shows the dentin, and P shows the periodontal ligament, and the bar next to the letter P in the figure shows the width of the periodontal ligament. The scale bar in the lower right of the figures shows 100 µm. The periodontal ligament of the group with stimulation was formed thicker than the group without stimulation, and ordered sequence of cells in the periodontal ligament region was recognized.
The result of measuring the width of the periodontal ligament on the CT image is also shown in Figure 7. The width of the periodontal ligament of the group with stimulation was 108.5 ± 30.6 µm (n = 33) and the width of the periodontal ligament of the group without stimulation was 68.7 ± 14.1 µm (n = 12) (Student's t-test, *p<0.0001).
From the above result, it was found that a regenerated tooth unit having sufficient periodontal ligament tissue is obtained by applying mechanical stimulation from the exterior when culturing a regenerated tooth germ in vivo.

### <Example 3: Jaw Bone Transplantation of Regenerated Tooth Unit Obtained by In Vivo Culture>

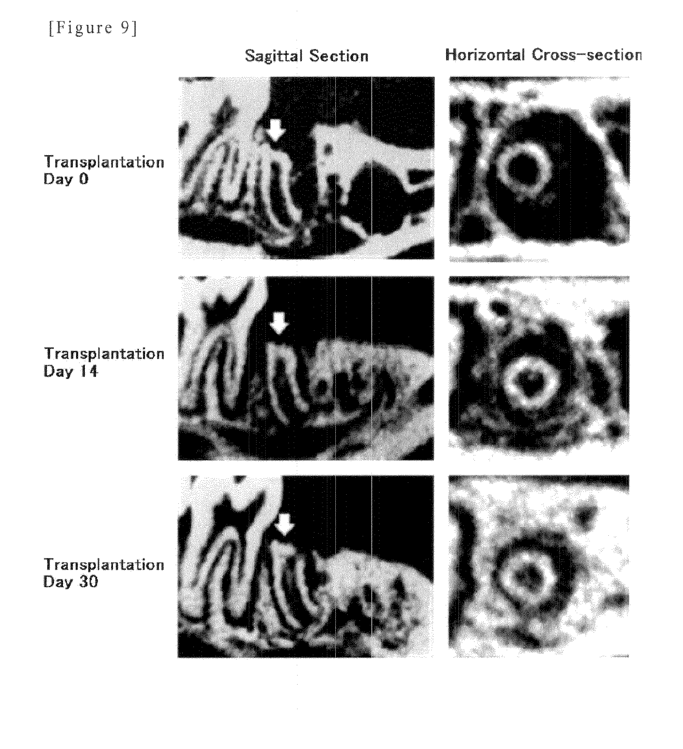

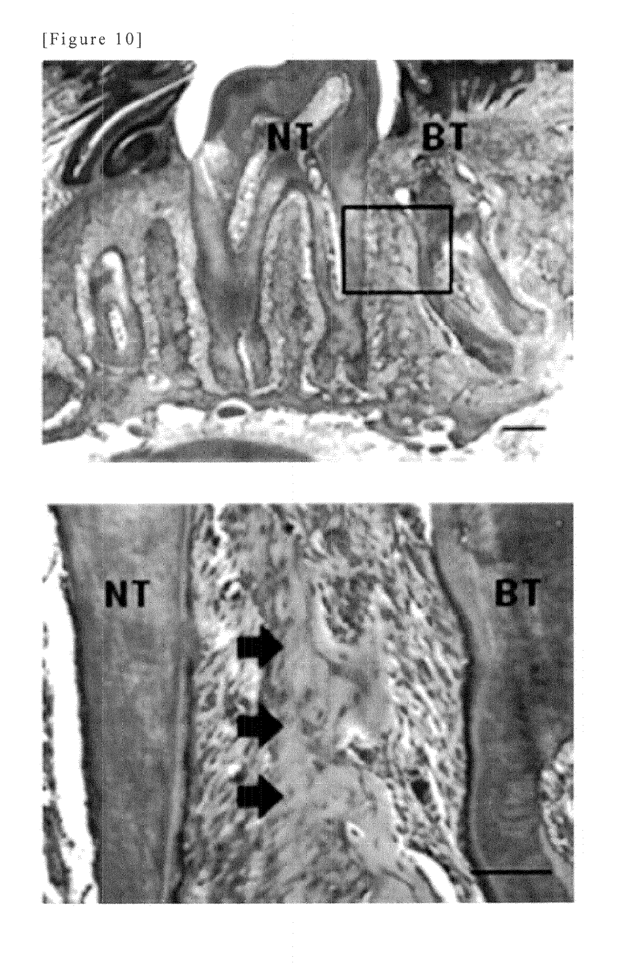

The first molar of the lower jaw of a 4 weeks-old C57BL/6 mouse was extracted, and 3 days of healing period was provided. Gingival incision/detachment of the same portion was then performed, the alveolar bone was cut with a dental micromotor (Viva-Mate Plus, Nakanishi Inc., Tokyo, Japan), and a transplantation fossa having a mesiodistal diameter of 1 mm and a buccolingual diameter of 0.8 mm was formed.
A regenerated tooth unit produced by culturing with a spacer for 30 days in the subrenal capsule according to Example 1 and applying mechanical stimulation during culture according to Example 2 was embedded in the jaw bone transplantation fossa, and the gingiva was sutured with an 8-0 nylon surgical suture (Bear Medic Corporation, Chiba, Japan).

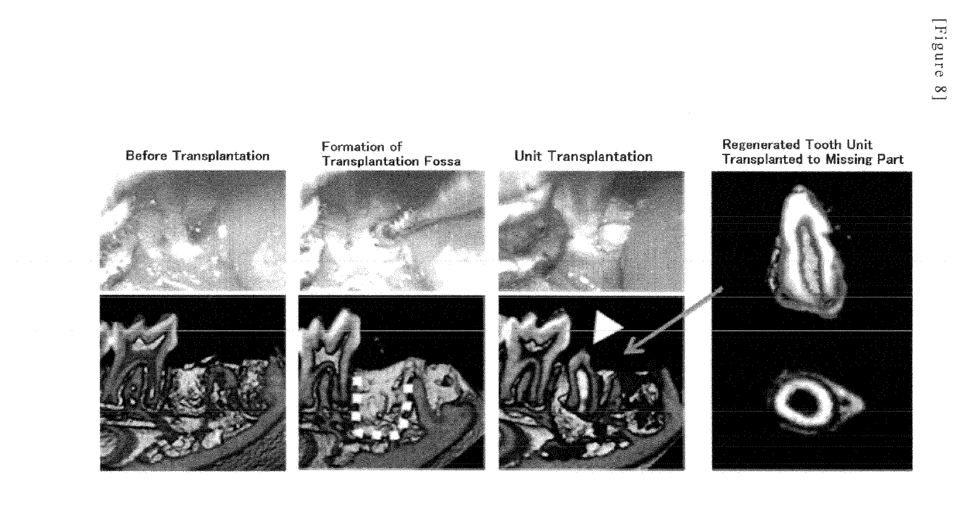

Figure 8 shows the appearance of the formation of the transplantation fossa and the transplantation of a regenerated tooth unit.
Micro CT images of a mouse with transplanted regenerated tooth unit were taken on transplantation Day 0, Day 14, and Day 30 with a method similar to above, and the attachment between the graft and the recipient alveolar bone was evaluated. The jaw bone comprising the graft was then fixed with 4 % PFA, decalcified with 10 % formic acid-sodium citrate decalcification solution, and embedded in paraffin, after which continuous sections 8 µm thick were created and histological evaluation was performed by HE staining.
The CT image is shown in Figure 9, and the HE staining image is shown in Figure 10.
As shown in Figure 9, partial attachment was observed on transplantation Day 14 between the graft and the recipient's second molar alveolar bone of the lower jaw, and bone attachment was observed in almost the entire circumference on transplantation Day 30. In the figure, the arrow shows the regenerated tooth unit.
As shown in Figure 10, the possibility that the alveolar bone of the interalveolar septum between the regenerated tooth and the second molar of the lower jaw are fused and that the regenerated tooth unit is integrated with the alveolar bone of the transplantation site via bone attachment was shown in the tissue image on transplantation Day 30. Also from tissue analysis, although calcification was observed on a part of the regenerated tooth dental pulp as an accidental symptom of transplantation (3 out of 16 cases), the periodontal ligament of the regenerated tooth unit was maintained after transplantation, and no bone adhesion was observed (0 out of 16 cases). In the figure, the arrow shows integration by bone attachment, the scale bar shows 100 µm, BT shows the regenerated tooth, and NT shows the natural second molar of the lower jaw. Consequently, it was shown that a regenerated tooth unit manufactured with the manufacturing method of the present invention is integrated well when transplanted to the jaw bone.

### <Example 4: Production of Multiple-teeth Regenerated Tooth Unit>

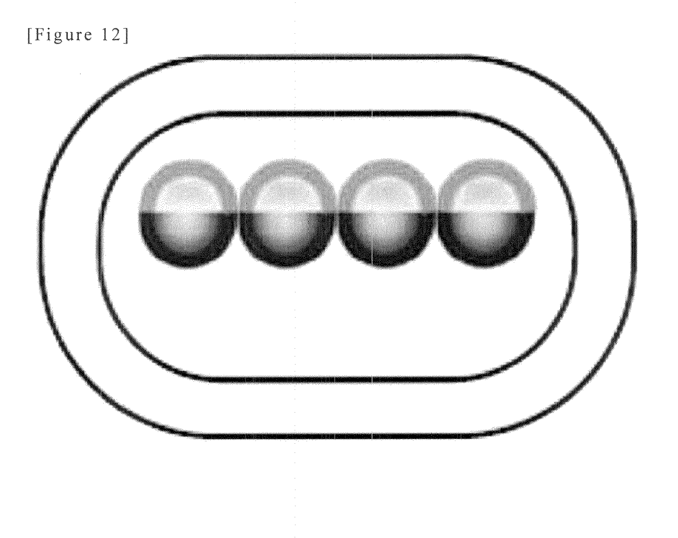

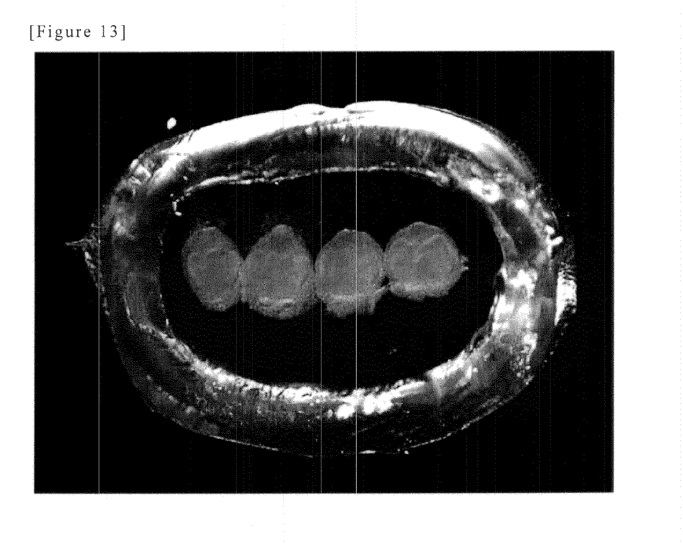

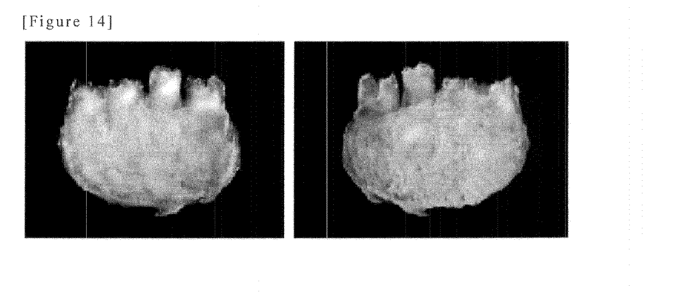

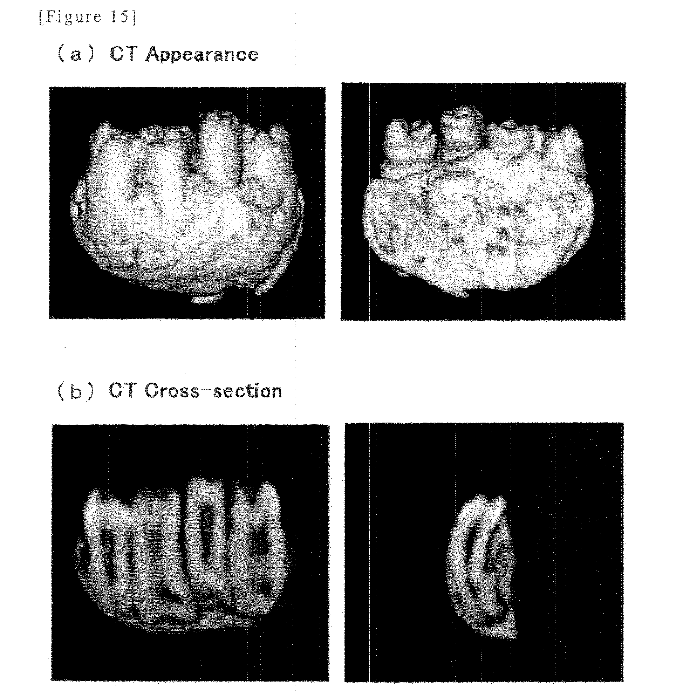

With the purpose producing a multiple-teeth regenerated tooth unit which is transplantable to a large tooth loss, in vivo culturing of a multiple regenerated tooth germs was performed in subrenal capsule transplantation with a spacer.
A micropipette chip (HRI-110 New Molecular Bio Products, San Diego, Ca, USA) was cut in a cylinder-shape, shaped so that the cross-sectional shape of the cylinder was a rounded rectangle having a major axis 3 mm/minor axis 2 mm/height 1.3 mm to employ as the spacer, and filled the interior with a collagen solution and used. Four regenerated tooth germs were disposed in contact with each other such that the height direction of the cylinder will be the thickness direction of the future regenerated tooth, and the minor axis direction of the rounded rectangle will be the tooth extension direction and the major axis direction of the rounded rectangle will be the dentition direction of teeth. At this time, the future tooth crown side of the regenerated tooth germ was disposed somewhat in proximity to the wall of one of the long sides of the rounded rectangle of the spacer cross-section in order to promote extension in the tooth root direction, and the collagen gel was hardened in a CO₂ incubator (SANYO Electric Co.,Ltd, Osaka, Japan) at 37 degrees. A schematic diagram of the state of disposing multiple regenerated tooth germs inside a spacer is shown in Figure 12, and the photograph thereof is shown in Figure 13. Figures 12 and 13 are the figure or the photograph of the cylindrical spacer viewed from the axial direction of the cylinder. Next, the spacer comprising a regenerated tooth germ was transplanted to the bilateral subrenal capsule of a 7 weeks-old C57BL/6 mouse. Transplantation to the subrenal capsule was performed by making a 5 mm incision in the renal capsule, detaching the renal capsule and the renal parenchymal, and inserting between them the spacer comprising multiple regenerated tooth germs.
A mass of multiple-teeth unit having multiple teeth was obtained by harvesting from the kidney 60 days after subrenal capsule transplantation. The stereophotograph of the multiple-teeth unit at 60 days after subrenal capsule transplantation is shown in Figure 14. When analyzed with micro CT (Rigaku Corporation, Tokyo, Japan), periodontal space around each tooth and interalveolar septum of the alveolar bone were observed. The CT appearance image and CT cross-sectional image for the multiple-teeth unit at 60 days after subrenal capsule transplantation are shown in Figure 15. Consequently, as one aspect of the manufacturing method of the present invention, it was shown that by disposing and culturing individual regenerated tooth germs which respectively grow into single teeth with the direction of the future occlusal surfaces aligned, a regenerated tooth unit in a state where the tooth crown-root direction of multiple individual teeth is aligned in one direction similarly to the innate tooth, and where multiple teeth is aligned in a row so as to form a dentition is obtained. In addition, as one aspect of the manufacturing method of the present invention, it was shown that a regenerated tooth unit produced by disposing numerous regenerated tooth germs exist with multiple teeth having normal tissue structure, and that the alveolar bone is formed as a monolith so that it covers the tooth root portion thereof.

### <Example 5: Transplantation of Multiple-teeth Unit to Edentulous Jaw Model>

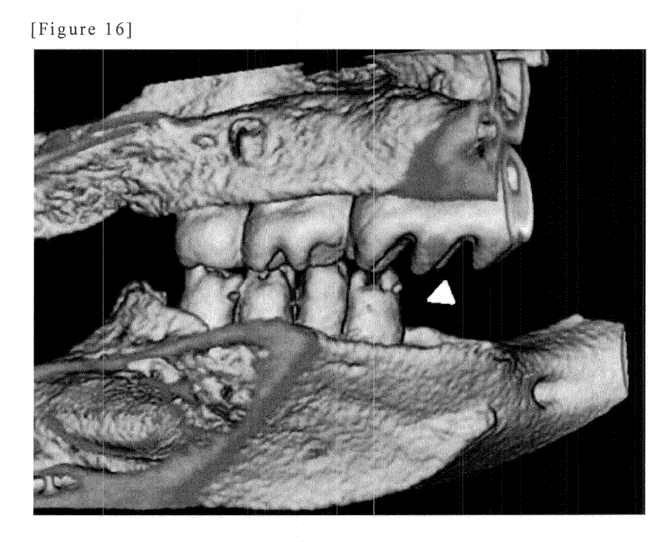

The first, second, and third molar of the lower jaw of a 4 weeks-old C57BL/6 mouse was extracted, an edentulous jaw model was generated, and 3 days of healing period was provided. Gingival incision/detachment of the same portion was then performed, the alveolar bone was cut with a dental micromotor (Viva-Mate Plus, Nakanishi Inc., Tokyo, Japan), and a transplantation fossa having a mesiodistal diameter of 3 to 4 mm and a buccolingual diameter of 1.2 mm was formed. A regenerated tooth unit produced under the mouse renal capsule was embedded in the jaw bone transplantation fossa, and the gingiva was sutured with an 8-0 nylon surgical suture (Bear Medic Corporation, Chiba, Japan).
Micro CT images were taken for the edentulous jaw model mouse with a multiple-teeth unit transplanted. The CT image on Day 1 after transplantation of multiple-teeth unit into the mouth is shown in Figure 16. From the observation in this Example, integration by bone attachment between the multiple-teeth unit alveolar bone and the recipient alveolar bone was suggested.
This shows that a regenerated tooth unit comprising multiple teeth compatible to the shape of the missing tooth site can be transplanted at once even when the missing tooth site is large, and it was suggested that there is good bone attachment between the periodontal tissue of the transplanted regenerated tooth unit and the jaw bone of the transplanted individual. In addition, bone integration was observed between the periodontal tissue of the transplanted regenerated tooth unit and the jaw bone of the transplanted individual in the CT image on Day 50 after transplantation.

## Claims

1. A method for manufacturing a regenerated tooth unit by culturing a regenerated tooth germ in vivo in the body of a mammal, comprising the steps of:
preparing a spacer;
disposing said regenerated tooth germ inside said spacer in vivo in the body of said mammal;
culturing said regenerated tooth germ in vivo in the body of said mammal;
wherein,
said spacer is configured so as to enable prevention of said regenerated tooth germ being extended to more than the maximum tolerance and such that the regenerated tooth germ disposed inside is communicatable with the exterior of the spacer.

2. The method for manufacturing a regenerated tooth unit according to claim 1, **characterized in that** said mammal is a non-human mammal.

3. The method for manufacturing a regenerated tooth unit according to any of claim 1 or claim 2, **characterized in that** said spacer is further configured so as to enable prevention of excessive pressure being applied to said regenerated tooth germ from a tissue inside the body of said mammal.

4. The method for manufacturing a regenerated tooth unit according to any one of claims 1 to 3, **characterized in that**
the step of disposing said regenerated tooth germ inside said spacer in vivo in the body of said mammal is a step of disposing so that the future occlusal surface site of the regenerated tooth germ will be faced in the vicinity of the inner wall of said spacer, and disposing so that the future root apex site extends towards a larger space in a direction opposite to the said inner wall of said spacer.

5. The method for manufacturing a regenerated tooth unit according to any one of claims 1 to 4, **characterized in that**
the step of disposing said regenerated tooth germ inside said spacer in vivo in the body of said mammal is disposing so that said regenerated tooth germ is supported by a support carrier that fills the interior of said spacer.

6. The method for manufacturing a regenerated tooth unit according to any one of claims 1 to 5, **characterized in that** said regenerated tooth germ is cultured and maintained by disposing a first cell assembly composed substantially of mesenchymal cells and a second cell assembly composed substantially of epithelial cells in close contact inside a support carrier, wherein at least either one of mesenchymal and epithelial cells is tooth germ-derived.

7. The method for manufacturing a regenerated tooth unit according to any one of claims 1 to 6, **characterized in** further comprising a step of applying mechanical stimulation from the exterior of said mammal when culturing said regenerated tooth germ.

8. The method for manufacturing a regenerated tooth unit according to claim 7, **characterized in that** said mechanical stimulation is vibration at about 20000 Hz to about 40000 Hz.

9. The method according to claim 1, wherein said spacer has an interior space which enables the development of a regenerated tooth unit having a desired regenerated tooth unit size.

10. The method according to claim 9, wherein said spacer is rough cylindrical, in which the height of the said cylinder is greater than the thickness of the desired regenerated tooth unit, and the inner diameter of the said cylinder is the approximate maximum tolerance of the length of the regenerated tooth unit.

11. The method according to claim 10, wherein said spacer is circular cylinder-shaped.

12. The method for manufacturing a regenerated tooth unit according to any one of claims 1 to 11, **characterized in that** said regenerated tooth germ comprises individually prepared multiple regenerated tooth germs.

13. The method for manufacturing a regenerated tooth unit according to any one of claims 1 to 12, **characterized in that** said regenerated tooth germ comprises individually prepared multiple regenerated tooth germs, and the individually prepared multiple regenerated tooth germs are disposed adjacent to each other inside said spacer.

14. The method for manufacturing a regenerated tooth unit according to any one of claims 1 to 13, **characterized in that** said regenerated tooth germ comprises individually prepared multiple regenerated tooth germs, and the individually prepared multiple regenerated tooth germs are disposed in rough parallel alignment in the axial direction that connects the center of the future occlusal surface site of each regenerated tooth germ and the center of the future root apex site inside said spacer.

15. A regenerated tooth germ composition for manufacturing a regenerated tooth unit by disposing inside a spacer and culturing in vivo in the body of a mammal, **characterized in that** said spacer is configured so as to enable prevention of said regenerated tooth germ being extended to more than the maximum tolerance and such that the regenerated tooth germ disposed inside is communicatable with the exterior of the spacer.

16. The regenerated tooth germ composition according to claim 15, **characterized in that** said regenerated tooth germ comprises individually prepared multiple regenerated tooth germs.

17. A spacer for manufacturing a regenerated tooth unit by placing and culturing a regenerated tooth germ in vivo in the body of a mammal, **characterized in that** the spacer is configured so as to enable prevention of said regenerated tooth germ being extended to more than the maximum tolerance and such that the regenerated tooth germ disposed inside is communicatable with the exterior of the spacer.

18. The spacer according to claim 17, **characterized in that** the regenerated tooth germ disposed inside said spacer comprises individually prepared multiple regenerated tooth germs.

19. A method for manufacturing a regenerated tooth unit comprising a step of culturing regenerated tooth germ in vivo in the body of a mammal, wherein mechanical stimulation is applied from the exterior of said mammal when culturing said regenerated tooth germ.

20. The method for manufacturing a regenerated tooth unit according to claim 19, **characterized in that** said mechanical stimulation is vibration at about 20000 Hz to about 40000 Hz.

21. The method for manufacturing a regenerated tooth unit according to claim 19 or claim 20, **characterized in that** said regenerated tooth germ is cultured and maintained by disposing a first cell assembly composed substantially of mesenchymal cells and a second cell assembly composed substantially of epithelial cells in close contact inside a support carrier, wherein at least either one of mesenchymal and epithelial cells is tooth germ-derived.

22. The method for manufacturing a regenerated tooth unit according to any one of claims 19 to 21, **characterized in that** said regenerated tooth germ comprises individually prepared multiple regenerated tooth germs.

23. A method for repairing a missing tooth of an animal, comprising
a step of transplanting a regenerated tooth unit manufactured with the method for manufacturing a regenerated tooth unit according to any one of claims 1 to 14 and claims 19 to 22 to said missing site.

24. The method for repairing a missing tooth of an animal according to claim 23, **characterized in that** said animal is a non-human mammal.

25. The method for repairing a missing tooth of an animal according to any one of claims 23 to 24, **characterized in that** said regenerated tooth unit comprises multiple teeth in which the tooth crown-root direction is aligned in rough parallel.

26. A regenerated tooth unit manufactured with the method for manufacturing a regenerated tooth unit according to any of claims 1 to 14 and claims 19 to 22.

27. A regenerated tooth unit manufactured with the method for manufacturing a regenerated tooth unit according to any of claims 1 to 14 and claims 19 to 22 for transplanting to the missing part in order to repair the missing tooth in the mouth of an animal.

28. The regenerated tooth unit according to claim 26 or claim 27, **characterized in that** it comprises multiple teeth in which the tooth crown-root direction is aligned in rough parallel.

29. The regenerated tooth unit according to claim 26 or claim 27, **characterized in that** it comprises multiple teeth in which the tooth crown-root direction is aligned in rough parallel, and the periodontal tissue is monolithically formed.
